Europäisches Patentamt

European Patent Office

Office européen des brevets

(11) Veröffentlichungsnummer: **0 327 495**
**A2**

(12) **EUROPÄISCHE PATENTANMELDUNG**

(21) Anmeldenummer: 89810055.7

(22) Anmeldetag: 24.01.89

(51) Int. Cl.⁴: **A 61 F 2/38**

(30) Priorität: 05.02.88 CH 415/88

(43) Veröffentlichungstag der Anmeldung:
09.08.89 Patentblatt 89/32

(84) Benannte Vertragsstaaten: **AT DE FR GB IT**

(71) Anmelder: **GEBRÜDER SULZER
AKTIENGESELLSCHAFT
Zürcherstrasse 9
CH-8401 Winterthur (CH)**

**Protek AG
Stadtbachstrasse 64
CH-3001 Bern (CH)**

(72) Erfinder: **Essinger, Jacques R., Dr., Dipl.-Phys.
Av. des Roses 28
CH-1009 Pully (CH)**

**Aubanica, Jean-Manuel, Prof. Dr.-méd.
Le Puget
F-13590 Meyreuil (FR)**

(54) Tibiateil für eine Kniegelenkprothese.

(57) Der Tibiateil (1) einer Kniegelenkprothese ist im Grundriss hufeisenähnlich ausgebildet, wobei eine Aussparung (6) zwei Lappen (4, 5) im dorsalen Bereich trennt; dabei erstreckt sich der laterale Lappen (5) weiter in Richtung dorsal als der mediale (4).

Die Aussparung (6), deren Mittelachse (8) gegenüber derjenigen (9) des Tibiateils (1) zur Angleichung an die Anatomie nach medial versetzt ist, ermöglicht einen ungestörten Verlauf der Kreuzbänder (7).

Der weiter nach hinten gezogene laterale Lappen (5) gewährleistet eine Auflage und Abstützung der lateralen Femurkondyle auch bei um 90° abgewinkeltem Gelenk mit extremer Aussenrotation des Unterschenkels.

Für einen den natürlichen Verhältnissen möglichst angepassten Verlauf des Patella-Sehne (13) ist der vorderer Rand (12) des Tibiateils (1) auf der lateralen Seite abgeflacht.

EP 0 327 495 A2

## Beschreibung

### Tibiateil für eine Kniegelenkprothese

Die Erfindung betrifft einen im Knochen verankerbaren Tibiateil für eine Kniegelenkprothese, der auf einem durchgehenden Plateau die tibialen Lagerflächen für beide Femurkondylen enthält.

Für Kniegelenkprothesen, bei denen die Lagerflächen für beide Femurkondylen in einem als Monoblock ausgebildeten Tibiateil vereinigt sind, sind die Tibiateile im Grundriss bisher symmetrisch zu einer sagittalen Mittelebene in Längsrichtung der Tibia ausgebildet worden (siehe z.B. EP-A-0 218 032), um diese Tibiateile für rechte und linke Kniegelenke einsetzen zu können.

Die Praxis hat gezeigt, dass derartige Konstruktionen in verschiedenen Punkten nicht optimal sind. So ist beispielsweise bei diesen Konstruktionen keine Möglichkeit gegeben, eine laterale Auflage der Femurkondylen auf dem Tibiateil zu erreichen, wenn das Gelenk etwa 90° abgewinkelt und der Unterschenkel stark nach auswärts (Aussenrotation) gedreht ist. Schwierigkeiten können sich bei den bisherigen Tibiateilen auch wegen des Platzbedarfes der Patella-Sehne und/oder der Kreuzbänder ergeben.

Aufgabe der Erfindung ist es daher, die geschilderten Nachteile bisheriger Tibiateile der vorstehend beschriebenen Art zu beseitigen und diese Teile besser an die anatomischen Gegebenheiten anzupassen.

Mit der Erfindung wird diese Aufgabe dadurch gelöst, dass der Tibiateil nach dorsal in zwei, durch eine Aussparung für die Kreuzbänder getrennte Lappen ausläuft, wobei der laterale Lappen sich in Richtung dorsal weiter erstreckt als der medial, dass ferner die vertikale Mittelebene der Aussparung relativ zur Mittelebene des Implantates nach medial versetzt ist, und dass schliesslich der vordere Rand des Tibiateils auf der lateralen Seite abgeflacht ist.

Der lateral in Richtung dorsal verlängerte Lappen ermöglicht eine Auflage der lateralen Femurkondyle auch bei starker Aussenrotation und abgewinkeltem Gelenk. Durch die Ausparung, deren Mittelebene zur Mittelebene des Implantats nach medial versetzt ist, wird dem Platzbedarf und dem Verlauf der Kreuzbänder Rechnung getragen, während die Abflachung der lateralen "Vorderseite" die Bewegungsfreiheit für die Patella-Sehne verbessert.

In Verbindung mit dem neuen Tibiateil sind am Femur besonders sogenannte Schlittenprothesen verwendbar oder auch getrennte unikondyläre Schalenprothesen. Der Tibiateil selbst kann in bekannter Weise ein- oder zweiteilig ausgeführt sein, d.h. als zweiteilige Konstruktion, beispielsweise aus einem metallischen Verankerungsteil und einem darin eingesetzten, die Lager- und Gleitflächen für den Femur tragenden Kunststoffkörper bestehen.

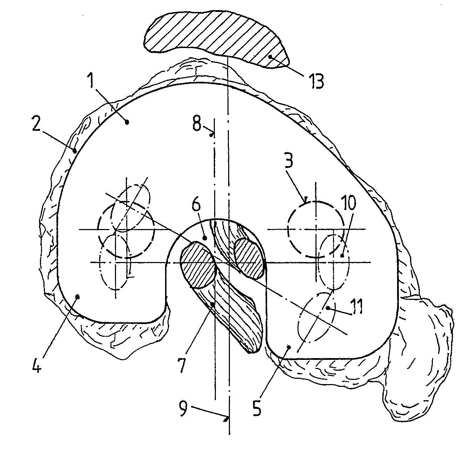

Im folgenden wird anhand eines Ausführungsbeispiels die Erfindung im Zusammenhang mit der Zeichnung näher erläutert.

Die einzige Figur zeigt schematisch eine Aufsicht in Richtung der Tibiaachse auf eine Ausführungsform des in einen Knochen eingesetzten neuen Tibiateils.

Der Tibiateil 1 ist in die operativ vorbereitete Tibia 2 eines rechten Beines in Richtung ihrer Achse eingesetzt und durch Verankerungszapfen 3 gehalten. Er hat annähernd die Form eines nach dorsal offenen Hufeisens, so dass je ein medialer und ein lateraler Lappen 4 und 5 entstehen. Zwischen diesen Lappen 4 und 5 ist eine Aussparung 6 vorhanden, durch die Kreuzbänder 7 verlaufen. Die vertikale Mittelebene 8 der Aussparung 6 ist dabei gegen diejenige 9 des Implantates 1 nach medial - also nach links -versetzt.

Weiterhin ist der laterale Lappen 5 (in der Figur rechts) gegenüber dem medialen Lappen 4 nach dorsal verlängert. Die durch strichpunktierte Ellipsen 10 bzw. 11 verdeutlichen, schematisch angedeutete, Auflagen für nicht gezeigte Femurkondylen eines gestrecktes Beines bzw. eines um etwa 90° abgewinkelten Kniegelenkes, bei dem der Unterschenkel stark nach auswärts verdreht ist; auf dem verlängerten lateralen Lappen 5 ergibt sich auch in der Extremstellung des abgewinkelten Unterschenkels - wie bei einem natürlichen Gelenk - eine Auflage zwischen Femur und Tibia.

Schliesslich ist der vordere Rand 12 des Tibiateils 1 auf der lateralen Seite abgeflacht, um einen möglichst natürlichen Verlauf der Patella-Sehne 13 zu ermöglichen.

### Patentansprüche

Im Knochen verankerbarer Tibiateil für eine Kniegelenkprothese, der auf einem durchgehenden Plateau die tibialen Lagerflächen für beide Femurkondylen enthält, **dadurch gekennzeichnet, dass** der Tibiateil (1) nach dorsal in zwei, durch eine Aussparung (6) für die Kreuzbänder (7) getrennte Lappen (4,5) ausläuft, wobei der laterale Lappen (5) sich in Richtung dorsal weiter erstreckt als der mediale (4), **dass ferner** die vertikale Mittelebene (8) der Aussparung (6) relativ zur Mittelebene (9) des Implantates (1) nach medial versetzt ist, **und dass schliesslich** der vordere Rand (12) des Tibiateiles (1) auf der lateralen Seite abgeflacht ist.